# EUROPEAN PATENT APPLICATION

(11) **EP 4 116 425 A1**
(43) Date of publication of application: **11.01.2023**
(21) Application number: 21305953.8
(22) Date of filing: 09.07.2021
(51) Int. Cl.: C12N 15/74

(54) **GENETICALLY MODIFIED BACTERIA AND USES THEREOF FOR THE INDUCIBLE EXPRESSION OF A NUCLEIC SEQUENCE OF INTEREST**

(71) Applicant: Institut national de recherche pour l'agriculture, l'alimentation et l'environnement, 75007 Paris (FR); Institut des sciences et industries du vivant et de l'environnement, 75005 Paris (FR)
(72) Inventor: GARDAN, Rozenn, 78350 JOUY-EN-JOSAS (FR); JUILLARD, Vincent, 92200 NEUILLY-SUR-SEINE (FR); MONNET, Véronique, 78180 MONTIGNY-LE-BRETONNEUX (FR)
(74) Representative: Gevers & Orès

(57) **Abstract**

The present invention relates to genetically modified bacteria capable of inducibly expressing a nucleic acid sequence of interest, genetic constructs, vectors and uses thereof.

## Description

The present invention relates to genetically modified bacteria capable of inducibly expressing a nucleic acid sequence of interest, genetic constructs, vectors and uses thereof.

Lactic acid bacteria (LAB) are attractive candidates for the production of molecules, in particular for therapeutic purposes. Indeed, they are bacteria with "GRAS" (Generally Recognized As Safe) status and, as such, they benefit from a healthy reputation. They are relatively easy to manipulate genetically and, therefore, potentially capable of expressing heterologous proteins through controlled expression systems. Moreover, they can be administered orally or intranasally and they can target many pathologies, including digestive ones, since they remain metabolically active during their passage through the digestive tract.

Among model LAB, *Lactococcus lactis* represents the reference bacterium in both academic studies and recombinant protein production platforms. For example, a controlled expression system based on the *NisRK* regulatory genes is widely used in *L. lactis.* This system involves a promoter positively controlled by the 2-component system NisRK and it is inducible via the nisin peptide. However, notwithstanding its usefulness, this expression system is not that much controlled, i.e. the promoter remains active at a significant level in the absence of the inducer. In contrast, only a few academic studies exist for *S*. *thermophilus* despite that this bacterium shows some advantages over *L. lactis,* such as its optimum growth temperature of 37°C or its natural competence for transformation. Indeed, this later property facilitates genetic manipulation and allows *S*. *thermophilus* to naturally incorporate exogenous DNA into its chromosome by homologous recombination. However, there is no satisfactory system for the controlled expression of proteins of interest in *S*. *thermophilus.*

There thus remains a genuine need for an effective system allowing the controlled expression of proteins of interest in lactic acid bacteria such as *S*. *thermophilus.*

The present invention is believed to meet such need by providing a new inducible expression system based on the use of a quorum sensing cellular communication mechanism discovered in streptococci.

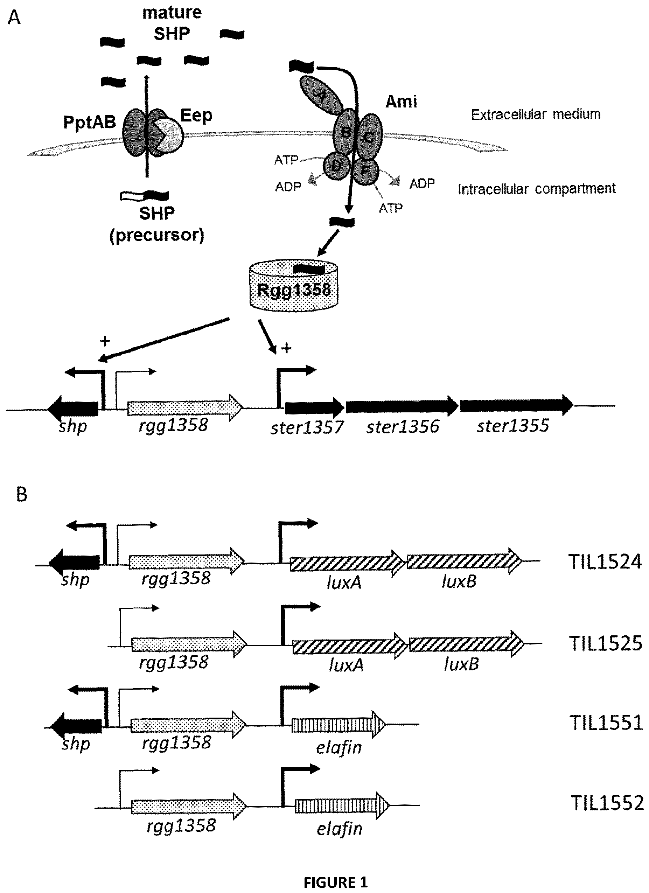

In some previous studies, the Inventors discovered a new quorum sensing system present in some *Streptococcus thermophilus* strains of industrial interest (Fleuchot et al., PLOS ONE 8(6):e66042, 2013). In the strain LMD-9, they showed that the *shp1358* gene encodes an auto-inductive small hydrophobic peptide (SHP) matured and secreted in culture supernatants. They then identified a membrane protease (Eep) as well as a peptide transporter (PptAB) essential for the production of a mature form of SHP in the extracellular environment. They also showed that an Ami oligopeptide transporter allows the import of the mature SHP. Finally, they demonstrated an interaction between the mature SHP peptide and a transcriptional regulator of the Rgg family and showed that the activity of Rgg is positively controlled by its interaction with the mature SHP. The SHP1358/Rgg1358 (STER_RS06695 according to the new NCBI nomenclature) complex controls the expression of two groups of target genes: the *shp1358* gene encoding the SHP precursor peptide and the ster_1357-ster_1355 operon (STER_RS10575, STER_RS06690 and STER_RS06685), involved in the production and secretion of a cyclic peptide, named streptide, the function of which remains unknown. A schematic representation of the quorum sensing mechanism involving the transcriptional regulator Rgg1358 and the hydrophobic peptide SHP1358 is shown in Figure 1.

In an original way, the Inventors designed a new inducible expression system based on the elements of this quorum sensing cellular communication mechanism. The expression system of the invention can be implemented in two variations: a complete version that is auto-regulated (self-induction) and an incomplete version that is regulated by the addition of a SHP peptide (external induction).

In the complete version, all the genes of the locus are present and/or have been introduced into the bacterium used to produce the protein. When the bacterium is cultivated in a medium devoid of hydrophobic peptides, the system is self-induced by its own SHP production and allows the strong expression of the protein of interest at the end of the exponential growth phase.

In the incomplete version, the bacterium does not contain the gene encoding the SHP peptide. In this version, the expression of the protein can be triggered during the growth of the bacterium by adding the SHP peptide to the culture medium. This strategy allows to disconnect the bacterial growth and the production of proteins of interest. This is particularly advantageous to avoid yield losses in the production of proteins affecting bacterial growth.

The Inventors validated both variations of the expression system with a marker protein (a luciferase) and a secreted protein of interest (elafin, which inhibits protease activity in patients with diseases inflammatory bowels).

Similar loci comprising a gene encoding a SHP, a gene encoding a regulator transcriptional Rgg and a promoter positively controlled by the complex SHP/Rgg can be found in many streptococci in one or several copies, and can therefore be used according to the present invention.

In an aspect, the present invention thus concerns a genetically modified bacterium capable of inducibly expressing a nucleic acid sequence of interest, wherein:
the genetically modified bacterium comprises a genetic construct comprising the nucleic acid sequence of interest operably linked to a promoter positively regulated by a protein complex formed by a small hydrophobic peptide called "SHP" and a regulator protein called "Rgg",
the promoter comprises a DNA-binding site selected from the group consisting of: GCA(A/T)ATA(T/G)GGGAAT(A/T) (SEQ ID NO: 1), AATTGC(G/T)TATAAGGGAAA (SEQ ID NO: 2), ATTTCATATCTTCAATTTT (SEQ ID NO: 3) and variants thereof having 1 or 2 substitutions,
the genetically modified bacterium expresses the Rgg protein,
the Rgg protein has at least 80% sequence identity with an amino acid sequence selected from the group consisting of: SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13.

A "genetically modified bacterium" as used herein, refers to a bacterium in which the genetic material of the bacterium has been altered using genetic engineering techniques. In particular, the term "genetically modified" refers to a bacterium with a modification, such as a gene knockout, or wherein an exogenous gene is introduced for expression of a protein. Those skilled in the art will appreciate that there are multiple ways to produce genetically modified bacteria such as by introducing heterologous nucleic acids and/or by generating mutations. In some embodiments a nucleic acid (e.g. a gene or portion thereof) is introduced into a bacterium using a suitable technique. In some embodiments a bacterium is transformed with a nucleic acid by a suitable technique. Non-limiting examples of suitable techniques for introducing a nucleic acid into a bacterium include electroporation, transduction (e.g., injection of a nucleic acid by a bacteriophage), microinjection, by inducing competence (e.g., by addition of alkali cations, cesium, lithium, polyethylene glycol, competence-inducing peptide or by osmotic shock), the like or combinations thereof. A nucleic acid can be introduced into a bacterium in the form of a linear or circular plasmid, for example. In some embodiments, transformed bacterium are selected for integration of a nucleic acid into the genome of the bacterium by using a suitable selection method (e.g. a selection marker).

As used herein, a "genetic construct" refers to an artificially-designed segment of DNA that is used to introduce genetic material into a target, namely a bacterium.

The "inducible expression" means that the protein can be regulated. By inducibly expressing a nucleic acid sequence of interest is meant herein that expression of a nucleic acid sequence of interest is at least in part influenced by at least one inducer, i.e. a SHP peptide. Hence, by regulating the amount of inducer that is administered to said expression system, one is capable of regulating the amount of expression of said nucleic acid sequence of interest. Preferably, expression of a nucleic acid sequence of interest is dependent on the presence of an inducer. This means that said nucleic acid is expressed in the presence of an inducer, while it is expressed to a significant lesser extent, or not expressed, in the absence of said inducer. In a preferred embodiment, said nucleic acid sequence is essentially not expressed in absence of said inducer.

In principle, any nucleic acid sequence of interest is inducibly expressed by a genetically modified bacterium according to the present invention. In particular, suitable applications for an inducible gene expression system according to the present invention are the production of a protein with therapeutic properties or health benefits or the production of proteins in the fields of white biotechnology and green chemistry. In an embodiment, the invention relates to a genetically modified bacterium as defined above, with the proviso that the nucleic acid sequence of interest does not encode a luciferase.

As used herein, "SHP" refers to a small hydrophobic peptide, i.e. a secreted peptide signaling molecule (or pheromone), which can regulate gene expression by a quorum-sensing mechanism. Upon transport into the bacterial cell, the SHP binds to and modulate activity of receptor proteins belonging to the Rgg family of transcription factors. Generally, the sequence of the SHP peptide is from 5 to 12 amino acids in length, has a D or E in position 1, has a G in positions 7, 8 and/or 9, has at least two I and contains at least 40% of amino acids I, L and V.

As used herein, "Rgg" refers to a regulatory protein, i.e. a transcription factor. Cytoplasmic transcription factors known as "Rgg proteins" are peptide pheromone receptors ubiquitous in Firmicutes, in particular in *Streptococcus* genus.

"Identity" with respect to percent amino acid sequence "identity" for peptides and proteins is defined herein as the percentage of amino acid residues in the candidate sequence that are identical with the residues in the target sequences after aligning both sequences and introducing gaps, if necessary, to achieve the maximum percent sequence identity. Percent sequence identity is determined by conventional methods. Briefly, two amino acid sequences are aligned to optimize the alignment scores using the ClustalW algorithm (Thompson et al., Nuc. Ac. Res. 22:4673-4680, 1994) and PAM250 weight matrix (Dayhoff et al., "Atlas of Protein Sequence and Structure." National Biomedical Research Foundation. Washington, DC 5:345-358, 1978) and default parameters as provided by the program MegAlign (DNASTAR, Inc.; Madison, WI). The percent identity is then calculated as: [Total number of identical matches x 100] divided by [length of the longer sequence + number of gaps introduced into the longer sequence in order to align the two sequences].

In an embodiment, the Rgg protein has at least 81%, 82%, 83%, 84%, 85%, 86%, 87%, 88%, 89%, 90%, 91%, 92%, 93%, 94%, 95%, 96%, 97%, 98%, 99% or 100% sequence identity with an amino acid sequence selected from the group consisting of: SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13.

**Table 1. Non-limitative examples of Rgg sequences identified in various S. thermophilus strains (LMD-9, CNRZ1066, CIRM956 and JIM8232).**

| | |
|---|---|
| SEQ ID NO: 4 | MKDYGKLFKKFRESRGLVLKDIAKLGVSVSHISRFERENTDLTISKFMSALNAINMPID EFMYAANDFQRDEFNEILEKISLFVSARNVSGMEKLLISQVEKIEKRETFYTLNTILIKIR LQDLSDKSYYDESDIQYLSDYLFSVEYWGCYELLLFANTVDVLNHQTMMLLSREMC KRSEFYKDLPNYRRLHSTMLLNCYIISIERDEYIDALYFEKQLNHSCFTETEIYEKLVFHY SKNLYELKKNRSNKAILEMKKCITAMKLANSENLAIKFENHLSGVLKM |
| SEQ ID NO: 5 | MDSNELLKLGEFYKELRIARKVKQKDVAKNLSVSQLSKFESGQTMLSADKMLEVIE GINLTFSEFGFALRDYKPTQHQLLMNKISKLSAENDKQSLLKLLDKYKESQLLYDKLNT LVIKNAIHLIDRDFTLSLDDSKFLSNYLFDIEDWTAYEIHLFGNTMPFLSDTDLLFLGKE LVSRSEIYSPLLDFRKALKYTYLNLISELVERRLTTHFNFFVNQLRMILDVFDTFEIILLNF LVLVYSYLIENSVNLEEVEQYILKVSEIEQLGLSKILEQRLSQYLRTNQKGER |
| SEQ ID NO: 6 | MKKFGEIFKKFRESRGLRLKDVAKAGISTSQLSRFEKGETDLTISKFMFILDEINMPIDE FMYAVHDFHRDELNELLSKVRHFVSTRDVEGMKKLLYSQMELEDRREKFHQINIILL KIRLQDLSGETYYTKDDLTYLTDYLFSVEYWGYYELLIFSNTLDVLNHDVFMVLAREM FRRSDFYKEIPNNRRLISTMLLNGYITCVEREKFMDALYFEKQLNQCFFIETEVYERLV FKYAQNLYQYKEDGNVKAIIEMKKCVGAMKLAGSHHLAKTYEGHLEKILASS |
| SEQ ID NO: 7 | MKEFGAIFKKFRKSRGIRLKDVAKAGISTSQLSRFEKGQTDLTITKFMLILDEINMPID EFMYAVHDFHRDDLNELLSKIRLFVSTHDIDGLKKLLSSQLESEPKREKFHHINTILLKI RLQDLSGESYYTQQDLADLTDYLFSVEYWGYYELLIFANTLDVLNHETLMVLAKEMS RRSDFYKEIPNNRRMISTMLLNVYITCIEREELIDALYFEKQLNQCFFIETEIYERLVFQY AQNYYQYKKEGNNQSIIEMKKYIGAMKLAGSEHIAKTYEAHLKKLIEENS |
| SEQ ID NO: 8 | MPIEEFVYAVRDFRRDDFNELLEKIRYLVSNRDIDGMKKLLISQMEKKDKKEPFASFN TILIKIRLQDLSGETYVTDQEIVTISDYLFSAEYWGYYELLLFMNTIDALNHNTMMILA KEMCHRLDFYRDIPNNRRLLATMLLNAYITCIERNEWMDALYFEKQLKYCNFSETE MYEKLVLHYANNLYDLKKNNNRHAVLEMRKCIAATKLVDSNQISIKFENYLQKVLDD MR |
| SEQ ID NO: 9 | MIKFLSALDFINVSIEEFMVIYRGYRNDSFEELLKKLHTLSLTKDAEGLRYLFNEVEELP NSTTKKIEKILILSCLSSVESENLITQSDLRFVTDYLFGIESWGYYELSILGNLAQYINGET LVSLGREVLRKTDYYYEVVRNRKLVIQLSINIMIRCIESEWFTKAAFFERHIDSLVNVES DMYEKVIFSYVQGYLKFAMGDSGGKQIMQESLEIFKLLKNTRLYKNYLEHFKSIVG |
| SEQ ID NO: 10 | MKSKLGSTLRKVRNGKQISICSVADEHLSKSQISRFERGESEISCIRLINILDKLHITLDEF LILHDEDYTKTESFANLVQYIRKQYSLQNINNIQSLLSDSSNYTLDPFEKTMVKSILHT MDSSIIPSDDELLQLADYLFKVEKWGYYEIILLGNCVRTIDYNSVFLLTKEMLNNYIYSS LNKTNKRIVTQLAINCLILSIDMEEFTNCFYLIDEIKALLDNELNFYEQTVFLYATGYFEF KRWQSTSGIEKMKQAIQVLDILGEDNLKLHYTIHFDKLINNK |
| SEQ ID NO: 11 | MNIQEKFKAIRKQRKLSLRDLANVAGSTSSISDFEKGKTNLSNDVLLQLLGFMVVEIN EIFEWSAFQDAEFLELMTQVENALQTQDITVLIQKRETLQEFFVAKNHYIYHIISLVLD VIISKLQEKEPTPEIISELTDYFFSLDYWTNLDVGLLGNIVHHFTTETLVLFTDSILKNTP NQLRNNLDRIKIDTVINCLSVLLARREKNASQELLQLLSRKQFPTYFTYEKLCISEFKAIY MYIWCDKEEADNIHQTILKTVSLLFTKDEANKWDDYFKEIISSADIKK |
| SEQ ID NO: 12 | MNTNESMDLGELFRELRIARGLKLKDVASDKLSVSQLSKFENGQTMLSSDKLLVAIS GIHMNFSEFGYALNNYQEPEFFKLGEKIAALHSKQDIDGLKKLLVTYKGCESFDVYNR LNLLVIKVAIHSLDNSSLITDEDKEFLTNYLYEIEAWTEYELYIFGNTMSILSDSDLIFLGK |
| | AFEERSKLYSSLTSHKKSAEIAFLNLILILIERKEIYYVRYFMSKLEEILNYQDMFAITYLH VLKQVSDYLNGDVKSIEKIDNDINMIEKLGNPIAASFLRINLQQFLH |
| SEQ ID NO: 13 | MLTKFGEIYKLFRESRKITLRDIEHRGISRSQLSRFEKGETDLTTTKFFLALEQINVPIEEF MYAVKDFKRDCFYQLIDEVKQCFLNRNVPKLHKMLIKRMENDDTSIFSTIEIIFLKIKL QELSGEEYFNDTDINRITDYLFGVNYWGMFEILLFGNIMYIFNYETFLLLSKELLHRTE FYHDIPSYRRVIASIALNAFIICIERDYLVDAKYFEKQISRFYFDESEIYERLIYTYARSFYE FKKKQTTQSILKMRKVIGFMRVVECEKLAERYEEHLNKILSSFSDDN |

In an embodiment, the promotor regulated by the protein complex SHP/Rgg comprises a DNA-binding site selected from the group consisting of: GCAAATAGGGGAATA (SEQ ID NO: 14), GCAAATAGGGGAATT (SEQ ID NO: 15), GCATATAGGGGAATA (SEQ ID NO: 16), GCATATAGGGGAATT (SEQ ID NO: 17), GCAAATATGGGAATA (SEQ ID NO: 18), GCAAATATGGGAATT (SEQ ID NO: 19), GCATATATGGGAATA (SEQ ID NO: 20), GCATATATGGGAATT (SEQ ID NO: 21), AATTGCGTATAAGGGAAA (SEQ ID NO: 22), AATTGCTTATAAGGGAAA (SEQ ID NO: 23) and ATTTCATATCTTCAATTTT (SEQ ID NO: 3).

In an embodiment, the SHP peptide is selected from the group consisting of: SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31 and SEQ ID NO: 32.

**Table 2. Non limitative examples of SHP peptides and their precursors identified in various S. thermophilus strains (LMD-9, CNRZ1066, LMG18311 and JIM8232). The sequence of the mature SHP peptide is indicated in bold in the sequence of the precursor.**

| **SHP (mature form)** | **SHP precursor** |
|---|---|
| **EGIIVIVVG** (SEQ ID NO: 24) | MKKQILLTLLLVVF**EGIIVIVVG** (SEQ ID NO: 33) |
| **EGIIVILVG** (SEQ ID NO: 25) | MKKQKLLLLVVLVC**EGIIVILVG** (SEQ ID NO: 34) |
| **EGIIVIGVG** (SEQ ID NO: 26) | MNKKALFSLLFVIL**EGIIVIGVG** (SEQ ID NO: 35) |
| **ESIIVIAVG** (SEQ ID NO: 27) | MNKESFLAILLLIF**ESIIVIAVG** (SEQ ID NO: 36) |
| **DIIIFPPFG** (SEQ ID NO: 28) | MKKVIAIFLFIQTVVVI**DIIIFPPFG** (SEQ ID NO: 37) |
| **DIIIIVGG** (SEQ ID NO: 29) | MEKVSKILPILILVM**DIIIIVGG** (SEQ ID NO: 38) |
| **EIIIIIAL** (SEQ ID NO: 30) | MNISIKRFLMILL**EIIIIIAL** (SEQ ID NO: 39) |
| **CIYTIVGGV** (SEQ ID NO: 31) | MKLLKIIVLLT**CIYTIVGGV** (SEQ ID NO: 40) |
| **CIYIIVGGV** (SEQ ID NO: 32) | MKLLKIIVLLT**CIYIIVGGV** (SEQ ID NO: 41) |

In an embodiment, the invention relates to a genetically modified bacterium as defined above, wherein:
- said Rgg protein is SEQ ID NO: 4 and said SHP peptide is SEQ ID NO: 24, or
- said Rgg protein is SEQ ID NO: 4 and said SHP peptide is SEQ ID NO: 25, or
- said Rgg protein is SEQ ID NO: 4 and said SHP peptide is SEQ ID NO: 26, or
- said Rgg protein is SEQ ID NO: 4 and said SHP peptide is SEQ ID NO: 27, or
- said Rgg protein is SEQ ID NO: 4 and said SHP peptide is SEQ ID NO: 28, or
- said Rgg protein is SEQ ID NO: 4 and said SHP peptide is SEQ ID NO: 29, or
- said Rgg protein is SEQ ID NO: 4 and said SHP peptide is SEQ ID NO: 30, or
- said Rgg protein is SEQ ID NO: 4 and said SHP peptide is SEQ ID NO: 31, or
- said Rgg protein is SEQ ID NO: 4 and said SHP peptide is SEQ ID NO: 32, or
- said Rgg protein is SEQ ID NO: 5 and said SHP peptide is SEQ ID NO: 24, or
- said Rgg protein is SEQ ID NO: 5 and said SHP peptide is SEQ ID NO: 25, or
- said Rgg protein is SEQ ID NO: 5 and said SHP peptide is SEQ ID NO: 26, or
- said Rgg protein is SEQ ID NO: 5 and said SHP peptide is SEQ ID NO: 27, or
- said Rgg protein is SEQ ID NO: 5 and said SHP peptide is SEQ ID NO: 28, or
- said Rgg protein is SEQ ID NO: 5 and said SHP peptide is SEQ ID NO: 29, or
- said Rgg protein is SEQ ID NO: 5 and said SHP peptide is SEQ ID NO: 30, or
- said Rgg protein is SEQ ID NO: 5 and said SHP peptide is SEQ ID NO: 31, or
- said Rgg protein is SEQ ID NO: 5 and said SHP peptide is SEQ ID NO: 32, or
- said Rgg protein is SEQ ID NO: 6 and said SHP peptide is SEQ ID NO: 24, or
- said Rgg protein is SEQ ID NO: 6 and said SHP peptide is SEQ ID NO: 25, or
- said Rgg protein is SEQ ID NO: 6 and said SHP peptide is SEQ ID NO: 26, or
- said Rgg protein is SEQ ID NO: 6 and said SHP peptide is SEQ ID NO: 27, or
- said Rgg protein is SEQ ID NO: 6 and said SHP peptide is SEQ ID NO: 28, or
- said Rgg protein is SEQ ID NO: 6 and said SHP peptide is SEQ ID NO: 29, or
- said Rgg protein is SEQ ID NO: 6 and said SHP peptide is SEQ ID NO: 30, or
- said Rgg protein is SEQ ID NO: 6 and said SHP peptide is SEQ ID NO: 31, or
- said Rgg protein is SEQ ID NO: 6 and said SHP peptide is SEQ ID NO: 32, or
- said Rgg protein is SEQ ID NO: 7 and said SHP peptide is SEQ ID NO: 24, or
- said Rgg protein is SEQ ID NO: 7 and said SHP peptide is SEQ ID NO: 25, or
- said Rgg protein is SEQ ID NO: 7 and said SHP peptide is SEQ ID NO: 26, or
- said Rgg protein is SEQ ID NO: 7 and said SHP peptide is SEQ ID NO: 27, or
- said Rgg protein is SEQ ID NO: 7 and said SHP peptide is SEQ ID NO: 28, or
- said Rgg protein is SEQ ID NO: 7 and said SHP peptide is SEQ ID NO: 29, or
- said Rgg protein is SEQ ID NO: 7 and said SHP peptide is SEQ ID NO: 30, or
- said Rgg protein is SEQ ID NO: 7 and said SHP peptide is SEQ ID NO: 31, or
- said Rgg protein is SEQ ID NO: 7 and said SHP peptide is SEQ ID NO: 32, or
- said Rgg protein is SEQ ID NO: 8 and said SHP peptide is SEQ ID NO: 24, or
- said Rgg protein is SEQ ID NO: 8 and said SHP peptide is SEQ ID NO: 25, or
- said Rgg protein is SEQ ID NO: 8 and said SHP peptide is SEQ ID NO: 26, or
- said Rgg protein is SEQ ID NO: 8 and said SHP peptide is SEQ ID NO: 27, or
- said Rgg protein is SEQ ID NO: 8 and said SHP peptide is SEQ ID NO: 28, or
- said Rgg protein is SEQ ID NO: 8 and said SHP peptide is SEQ ID NO: 29, or
- said Rgg protein is SEQ ID NO: 8 and said SHP peptide is SEQ ID NO: 30, or
- said Rgg protein is SEQ ID NO: 8 and said SHP peptide is SEQ ID NO: 31, or
- said Rgg protein is SEQ ID NO: 8 and said SHP peptide is SEQ ID NO: 32, or
- said Rgg protein is SEQ ID NO: 9 and said SHP peptide is SEQ ID NO: 24, or
- said Rgg protein is SEQ ID NO: 9 and said SHP peptide is SEQ ID NO: 25, or
- said Rgg protein is SEQ ID NO: 9 and said SHP peptide is SEQ ID NO: 26, or
- said Rgg protein is SEQ ID NO: 9 and said SHP peptide is SEQ ID NO: 27, or
- said Rgg protein is SEQ ID NO: 9 and said SHP peptide is SEQ ID NO: 28, or
- said Rgg protein is SEQ ID NO: 9 and said SHP peptide is SEQ ID NO: 29, or
- said Rgg protein is SEQ ID NO: 9 and said SHP peptide is SEQ ID NO: 30, or
- said Rgg protein is SEQ ID NO: 9 and said SHP peptide is SEQ ID NO: 31, or
- said Rgg protein is SEQ ID NO: 9 and said SHP peptide is SEQ ID NO: 32, or
- said Rgg protein is SEQ ID NO: 10 and said SHP peptide is SEQ ID NO: 24, or
- said Rgg protein is SEQ ID NO: 10 and said SHP peptide is SEQ ID NO: 25, or
- said Rgg protein is SEQ ID NO: 10 and said SHP peptide is SEQ ID NO: 26, or
- said Rgg protein is SEQ ID NO: 10 and said SHP peptide is SEQ ID NO: 27, or
- said Rgg protein is SEQ ID NO: 10 and said SHP peptide is SEQ ID NO: 28, or
- said Rgg protein is SEQ ID NO: 10 and said SHP peptide is SEQ ID NO: 29, or
- said Rgg protein is SEQ ID NO: 10 and said SHP peptide is SEQ ID NO: 30, or
- said Rgg protein is SEQ ID NO: 10 and said SHP peptide is SEQ ID NO: 31, or
- said Rgg protein is SEQ ID NO: 10 and said SHP peptide is SEQ ID NO: 32, or
- said Rgg protein is SEQ ID NO: 11 and said SHP peptide is SEQ ID NO: 24, or
- said Rgg protein is SEQ ID NO: 11 and said SHP peptide is SEQ ID NO: 25, or
- said Rgg protein is SEQ ID NO: 11 and said SHP peptide is SEQ ID NO: 26, or
- said Rgg protein is SEQ ID NO: 11 and said SHP peptide is SEQ ID NO: 27, or
- said Rgg protein is SEQ ID NO: 11 and said SHP peptide is SEQ ID NO: 28, or
- said Rgg protein is SEQ ID NO: 11 and said SHP peptide is SEQ ID NO: 29, or
- said Rgg protein is SEQ ID NO: 11 and said SHP peptide is SEQ ID NO: 30, or
- said Rgg protein is SEQ ID NO: 11 and said SHP peptide is SEQ ID NO: 31, or
- said Rgg protein is SEQ ID NO: 11 and said SHP peptide is SEQ ID NO: 32, or
- said Rgg protein is SEQ ID NO: 12 and said SHP peptide is SEQ ID NO: 24, or
- said Rgg protein is SEQ ID NO: 12 and said SHP peptide is SEQ ID NO: 25, or
- said Rgg protein is SEQ ID NO: 12 and said SHP peptide is SEQ ID NO: 26, or
- said Rgg protein is SEQ ID NO: 12 and said SHP peptide is SEQ ID NO: 27, or
- said Rgg protein is SEQ ID NO: 12 and said SHP peptide is SEQ ID NO: 28, or
- said Rgg protein is SEQ ID NO: 12 and said SHP peptide is SEQ ID NO: 29, or
- said Rgg protein is SEQ ID NO: 12 and said SHP peptide is SEQ ID NO: 30, or
- said Rgg protein is SEQ ID NO: 12 and said SHP peptide is SEQ ID NO: 31, or
- said Rgg protein is SEQ ID NO: 12 and said SHP peptide is SEQ ID NO: 32, or
- said Rgg protein is SEQ ID NO: 13 and said SHP peptide is SEQ ID NO: 24, or
- said Rgg protein is SEQ ID NO: 13 and said SHP peptide is SEQ ID NO: 25, or
- said Rgg protein is SEQ ID NO: 13 and said SHP peptide is SEQ ID NO: 26, or
- said Rgg protein is SEQ ID NO: 13 and said SHP peptide is SEQ ID NO: 27, or
- said Rgg protein is SEQ ID NO: 13 and said SHP peptide is SEQ ID NO: 28, or
- said Rgg protein is SEQ ID NO: 13 and said SHP peptide is SEQ ID NO: 29, or
- said Rgg protein is SEQ ID NO: 13 and said SHP peptide is SEQ ID NO: 30, or
- said Rgg protein is SEQ ID NO: 13 and said SHP peptide is SEQ ID NO: 31, or
- said Rgg protein is SEQ ID NO: 13 and said SHP peptide is SEQ ID NO: 32.

In an embodiment, the invention relates to a genetically modified bacterium as defined above, wherein the bacterium expresses a native form of the SHP peptide.

As used herein, the "precursor" or "native form" of the SHP peptide refers to a full-length precursor peptide comprising the sequence of the SHP peptide. The SHP peptide is then produced after a cleavage by a bacterial protease, in particular by a membrane protease. When the genetically modified bacterium expresses the native form of the peptide, the expression is auto inducible.

In an embodiment, the invention relates to a genetically modified bacterium as defined above, wherein the bacterium does not express a native form of the SHP peptide.

When the genetically modified bacterium does not express the native or mature form of the peptide, the expression is externally inducible. In such an embodiment, the SHP peptide must be added to the culture medium of the bacterium to induce the expression of the nucleic acid sequence of interest.

In an embodiment, the invention relates to a genetically modified bacterium as defined above, wherein the genetic construct further comprises a nucleic sequence encoding the Rgg protein.

In an embodiment, the invention relates to a genetically modified bacterium as defined above, wherein the genetic construct further comprises a nucleic sequence encoding the SHP peptide.

In an embodiment, the invention relates to a genetically modified bacterium as defined above, wherein the genetic construct is plasmid-borne or is integrated in the genome of said bacterium.

In an embodiment, the genetically modified bacterium according to the invention is a lactic acid bacterium, preferably from the genus *Streptococcus.*

In an embodiment, the genetically modified bacterium according to the invention is selected from the group consisting of: *S. thermophilus, S. salivarius, S. agalactiae, S. pyogenes, S*. *pneumoniae, S. suis, S. mutans* and *S. mitis,* preferably *S*. *thermophilus* or *S*. *salivarius.*

In an embodiment, the genetically modified according to the invention is a non-pathogenic streptococcus, such as *S*. *thermophilus* or *S*. *salivarius.*

In an embodiment, the genetically modified according to the invention is a pathogenic streptococcus, such as *S*. *agalactiae, S. pyogenes, S. pneumoniae, S. mitis, S. mutans* and *S. mitis.* In an embodiment, the genetically modified bacterium according to the invention is a probiotic strain.

In an embodiment, the invention relates to a genetically modified bacterium as defined above, with the proviso that the genetically modified bacterium is not, or is not derived from, *S*. *thermophilus* LMD-9 or *S*. *thermophilus* LMG18311/ATCC BAA-250.

In an embodiment, the genetically modified bacterium according to the invention is from the genus *Enterococcus.*

In an embodiment, the genetically modified bacterium according to the invention is selected from the group consisting of *E. faecalis* and *E. faecium,* preferably *E. faecalis.*

In some cases, the production of proteins of interest by bacteria can be rendered difficult due to the surface proteolysis, especially in Gram positive bacteria. Indeed, surface proteolysis leads to a degradation of these proteins during and/or after their export, leading to a drop in yield and/or a deterioration of the structure and activity of the protein of interest.

In an embodiment, the invention thus relates to a genetically modified bacterium as defined above, wherein the bacterium has no or low surface proteolytic activity.

Preferably, a bacterium having no or low surface proteolytic activity is a bacterium of the species *Streptococcus thermophilus,* wherein an endogenous surface protease homologous to the protein designated STR_RS07745 in *Streptococcus thermophilus* CNRZ1066 has a reduced or abolished expression and/or activity. Bacteria having no or low surface proteolytic activity to be used in the present invention and method to produce such bacteria are described in details in PCT/EP2021/055561.

Unexpectedly, the Inventors also observed that, in some cases, a residual expression of the protein of interest can be detected even in the absence of the peptide SHP. This leak of the controlled system can be explained by the fact that other loci encoding similar quorum sensing components, including similar SHP peptides, are naturally present in many streptococci.

In order to overcome this issue, the Inventors have further developed an improved version of the expression system that avoids any expression of the sequence of interest unless the peptide SHP is added to the culture medium.

In such embodiments, the ABC-type transporter that enables export of the SHP peptide to the extracellular medium and/or the membrane protease that enables cleavage of the native form of the SHP peptide, are not functional or inactivated. In these embodiments, the similar SHP peptides that can be naturally produced by the bacterium are not matured and thus, cannot affect the expression system of the present invention.

In an embodiment, the invention relates to a genetically modified bacterium as defined above, wherein an ABC-type transporter called "PptAB", which enables export of the SHP peptide to the extracellular medium, is not functional or is inactivated.

In an embodiment, said PptAB comprises or consists in the amino acid sequence SEQ ID NO: 42 and SEQ ID NO: 43 or any variants thereof.

**Table 3. Sequences of the subunits of PptAB of S. thermophilus strain CNRZ1066.**

| | |
|---|---|
| PptA SEQ ID NO: 42 | MLKLEKVTGGYANIPVLKDVTFEVADGELVGLIGLNGAGKSTTINEIIGLLTPYGGQIAID GLTIQQDPASYRQKIGYIPETPSLYEELTLREHLETVLMAYGLDVTEGMARADKYLKLFRL DEKLDWFPTQFSKGMKQKVMIICAFIVNPSLFIVDEPFLGLDPLAISDLIELLAEEKANGK SILMSTHVLDSAEKMCDRFVILHHGQILAQGTIDELRQTFGDSSASLNDIYMQLTKGELS |
| PptB SEQ ID NO: 43 | MIAELFKERSRRLTTRCAKYSRYVFNDHFILVLLFLLGFVLVQYSQLLRHFPKNPWAIILGL LVLCLLLPFWGNIATYLEPADKHYLLVKEEEVLDHIKKATGCAFRFWVLIQTLIFILVVPLFL ALGLPVWGVVLAVAMAILKYFIFQKKAQPFYKQSGLNWTDAIAAENKRQQSILKFFSL FTNVKGITSSVKRRGYLDGILKRTKKDKKYTWYNLYLRAFLRSGDYFALSLRLFALTLLVIFL VPEKWLAMVLVVVFDYLLLFQLTALKSHFAYQRMANLMPIGKDMQVSNLKRLINQIVL VMTLIQALCLFDLKFSLILIGVMLVLSLVYLPAKLKKMID |

In an embodiment, the invention relates to a genetically modified bacterium as defined above, wherein a transmembrane protease called "Eep", which enables cleavage of the native form of the SHP peptide, is not functional or is inactivated.

In an embodiment, said Eep comprises or consists in the amino acid sequence SEQ ID NO: 44 or any variants thereof.

**Table 4. Sequence of Eep of S. thermophilus strain CNRZ1066.**

| | |
|---|---|
| Eep SEQ ID NO: 44 | MKAIITFLLIFCVIVVFHEFGHFFFAKRSGILVREFAIGMGPKIFAHTGKDGTVYTIRILPL GGYVRMAGWGEDTTEIKTGSPASLTLGKDGKVRRINLSDRQVDQTALPMNVTAYDL EDKLTITGLVLGETKTYEVDHDATIVEEDGTELRIAPKDVQYQNASIWGRLITNFAGPM NNFILGVLVFIILAFVQGGVQDTSTNLIQVTNGGAAQVSGLKTGDAIVAINKDKVTDW DSLKEALRENTQKFSKGDSLSVTVKRSNGQEETISVKPQESQGSYFLGVSPVLKTGLKD KIFGGFQMAWEGATAILATLKGLITNFSLNKLGGPVAMFQMSAQASESGLISILDLMG MLSINLGIFNLIPIPALDGGKIVMNIIEAIRRKPLNQKIESYITLAGVAVMVVLMIAVTW NDIMRAFF |

In another aspect, the invention relates to a genetic construct comprising:
- a nucleic acid sequence of interest operably linked to a promoter that is positively regulated by a protein complex formed by a small hydrophobic peptide called "SHP" and a regulator protein called "Rgg", said promoter comprising a DNA-binding site selected from the group consisting of: GCA(A/T)ATA(T/G)GGGAAT(A/T) (SEQ ID NO: 1), AATTGC(G/T)TATAAGGGAAA (SEQ ID NO: 2), ATTTCATATCTTCAATTTT (SEQ ID NO: 3) and variants thereof having 1 or 2 substitutions,
   and at least one genetic element selected from the group consisting of:
   - a nucleic acid sequence encoding the Rgg protein, said Rgg protein having at least 80% sequence identity with an amino acid sequence selected from the group consisting of: SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13,
   - a nucleic acid sequence encoding the SHP peptide, said SHP peptide being selected from the group consisting of: SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31 and SEQ ID NO: 32.

In an embodiment, the invention relates to a genetic construct as defined above, wherein said genetic construct further comprises genetic elements allowing the integration of the nucleic acid sequence of interest into the genome of a bacterium.

In an embodiment, the invention relates to a genetic construct as defined above, with the proviso that the nucleic acid sequence of interest does not encode a luciferase

In another aspect, the invention relates to a genetic vector comprising a genetic construct as defined above.

In an embodiment, the invention relates to a genetic vector as defined above, said vector being a plasmid, in particular a replicative plasmid or an integrative plasmid.

In another aspect, the invention relates to a the use of a genetically modified bacterium as defined above, a genetic construct as defined above or a genetic vector as defined above, for producing a protein of interest, said protein of interest being encoded by the nucleic acid sequence of interest.

In another aspect, the invention relates to a method for producing a protein of interest comprising a step of culturing a genetically modified bacterium as defined above.

In another aspect, the invention relates to a method for producing a protein of interest comprising a step of genetically modifying a bacterium with a genetic construct or a genetic vector as defined above.

In an embodiment, the invention relates to a method as defined above, wherein said genetically modified bacterium is cultivated in a culture medium supplemented with the SHP peptide, preferably during the exponential phase of growth.

In an embodiment, the invention relates to a method as defined above, wherein said genetically modified bacterium is cultivated in a culture medium in the absence of SHP.

In an embodiment, the invention relates to a method as defined above, with the proviso that the genetically modified bacterium is not, or is not derived from, *S*. *thermophilus* LMD-9 or *S*. *thermophilus* LMG18311/ATCC BAA-250.

In another aspect, the invention relates to a genetic vector for inducibly expressing a nucleic acid sequence of interest comprising:
- a promoter that is positively regulated by a protein complex formed by a small hydrophobic peptide called "SHP" and a regulator protein called "Rgg", said promoter comprising a DNA-binding site selected from the group consisting of: GCA(A/T)ATA(T/G)GGGAAT(A/T) (SEQ ID NO: 1), AATTGC(G/T)TATAAGGGAAA (SEQ ID NO: 2), ATTTCATATCTTCAATTTT (SEQ ID NO: 3) and variants thereof having 1 or 2 substitutions, and being located in 5' of a cloning site allowing the insertion of the nucleic acid sequence of interest,
   and at least one genetic element selected from the group consisting of:
   - a nucleic acid sequence encoding the Rgg protein, said Rgg protein having at least 80% sequence identity with an amino acid sequence selected from the group consisting of: SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13, and
   - a nucleic acid sequence encoding the SHP peptide, said SHP peptide being selected from the group consisting of: SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31 and SEQ ID NO: 32.

In another aspect, the invention relates to a kit for inducibly expressing a nucleic acid sequence of interest comprising:
- a genetic vector as defined above, and at least one component selected from:
- a SHP peptide, and
- a bacterium, preferably a *Streptococcus.*

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

**Figure 1**. Schematic representation of the SHP/Rgg₁₃₅₈ quorum-sensing mechanism of *S*. *thermophilus* LMD-9 and of the genetic constructs used in the study. (A) The quorum-sensing signal is encoded by the *shp* gene. The pheromone, a small hydrophobic peptide called mature SHP, is produced by C-terminal cleavage of a precursor SHP and exported, two processes that involve the endopeptidase Eep and the transporter PptAB, respectively. At high cell density, the secreted SHP is reimported into the cell by the Ami transporter. Intracellular mature form SHP then interacts with the regulatory protein Rgg₁₃₅₈. Binding of the SHP/Rgg₁₃₅₈ complex to the promoter region of the two target genes, *shp* and *ster_1357,* leads to the activation of their transcription. (B) DNA fragments encoding the complete or incomplete SHP/Rgg₁₃₅₈ system fused to the *luxAB* or elafin encoding gene are presented with the number of the corresponding strains on the right.

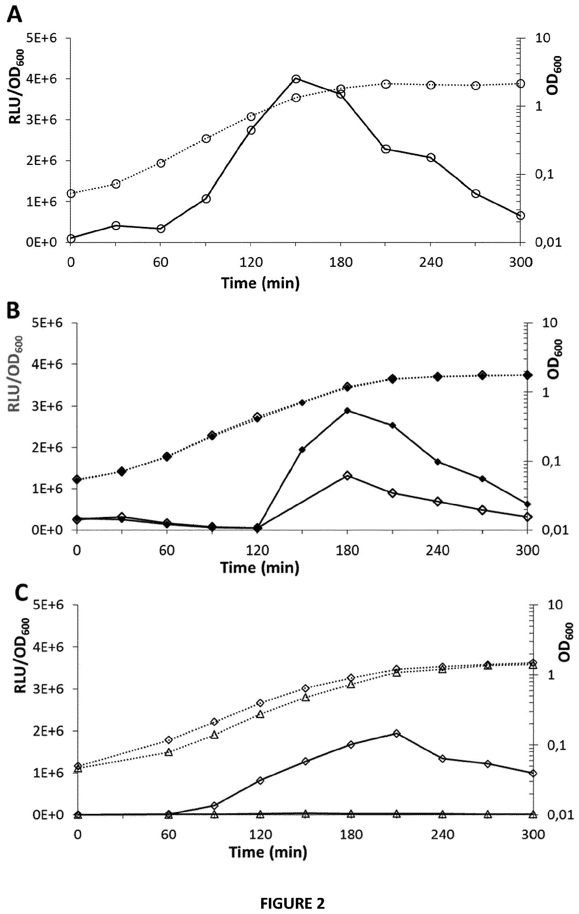

**Figure 2****.** Growth and luciferase activity levels for the *S*. *thermophilus* strains (A) TIL1524 strain (*blp*::*shp-rgg*₁₃₅₈-P*₁₃₅₇-luxAB*) grown in CDM (O), (B) TIL1525 strain (*blp*::*rgg*₁₃₅₈*-*P*₁₃₅₇-luxAB*) grown in CDM (0) or in CDM with synthetic SHP added 2h after the beginning of the growth culture at a final concentration of 1 µM (◆) and (C) TIL1525 strain (◊) and TIL 1566 strain (*blp*::*rgg*₁₃₅₈-P*₁₃₅₇-luxAB* Δ*pptAB*) (Δ) grown in CDM. The growth curves (OD₆₀₀) are depicted using dotted lines, and the relative levels of luciferase activity (RLU/OD₆₀₀) are depicted using black solid lines. Data shown are representative of three independent experiments.

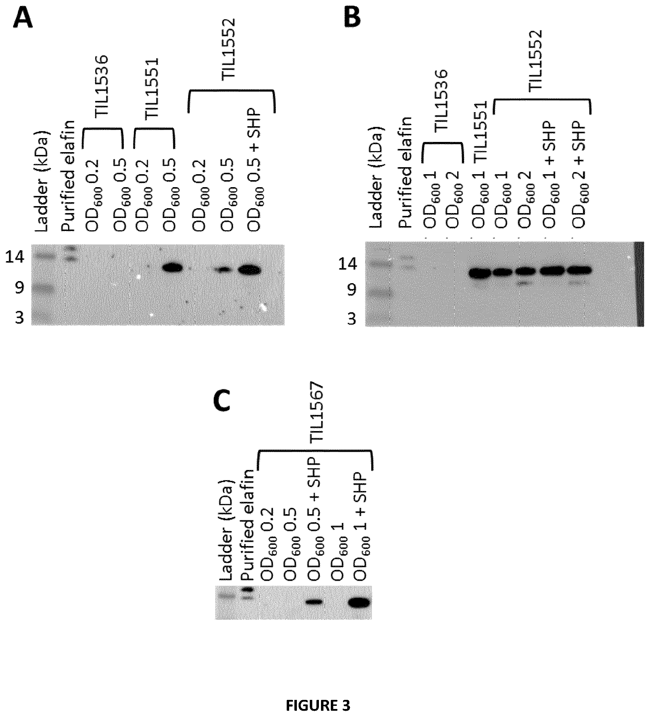

**Figure 3**. Production of elafin detected by Western blot in the supernatant of different *S*. *thermophilus* strains. (A) At OD₆₀₀ 0.2 and 0.5 for strain TIL1536 (Δ*htrA*::*aphA3* Δ*sepM*::*spec*), TIL1551 (*b*/*p::shp-rgg₁₃₅₈*-P_{ster*1357*}-*e*/*afin-P32cm* Δ*htrA*::*aphA3* Δ*ywdF*::*spec*) and TIL1552 (*blp::rgg₁₃₅₈*-P_{ster*1357*}-*elafin- P32cm* Δ*htrA*::*αphA3* Δ*ywdF*::*spec).* (B) At OD₆₀₀ 1 and 2 for strain TIL1536, TIL1551 and TIL1552. (C) At OD₆₀₀ 0.2, 0.5 and 1 for strain TIL1567 (*blp::rgg₁₃₅₈*-P_{ster*1357*}-*luxAB-aphA3* Δ*htrA*::*αphA3* Δ*ywdF*::*spec* Δ*pptAB::erm*). Purified elafin was used as a positive control and strain TIL1536 as negative control (elafin non-producing strain). Induction of the production of elafin with the addition of synthetic SHP in the culture medium is mentioned with: +SHP.

### EXAMPLES

### MATERIALS AND METHODS

### Bacterial strains and growth conditions

*S*. *thermophilus* wild-type strain LMD-9 (Makarova et al., Proc. Natl. Acad. Sci. U S A 103(42):15611-6, 2006) was used as a chromosomal DNA donor to retrieve the shp/rgg1358 system by PCR (Figure 1) and wild-type strain CNRZ1066 (Bolotin et al., Nat. Biotechnol. 22:1554-1558, 2004) was used as a chassis for the different genetic constructs. The *S*. *thermophilus* strains used in this study are listed in Table 5. *S*. *thermophilus* strains were grown at 42°C in either M17 medium (Difco) supplemented with 10 g I⁻¹ lactose or in a chemically defined medium (CDM) (Letort C & Juillard V, J. Appl. Microbiol. 91:1023-1029, 2001). *Escherichia coli* strains TG1*repA+* (Gardan et al., J. Bacteriol. 191:4647-4655, 2009) or TOP10 (Thermo Fischer Scientific) and *Lactococcus lactis* strain MG1363 (Gasson MJ, J. Bacteriol. 154:1-9, 1983) were used as hosts for the cloning experiments. *E. coli* strains were grown in Luria-Bertani broth with shaking at 30°C for strain TOP10 and at 37°C for strain TG1*repA+. L. lactis* strains were grown at 30°C in M17 medium (Difco) supplemented with 10 g I⁻¹ glucose. Agar (1.5%) was added to the media as needed. When required, antibiotics were added to the media at the following final concentrations: erythromycin at 150 µg ml⁻¹ for *E. coli* and at 5 µg ml⁻¹ for *S*. *thermophilus* and *L. lactis,* kanamycin at 1 mg ml-1 for *S*. *thermophilus,* spectinomycin at 150 µg ml⁻¹ for *S*. *thermophilus* and chloramphenicol at 5 µg ml⁻¹ for *E. coli, S. thermophilus* and *L. lactis.*

**Table 5. Streptococcus thermophilus strains used in this study. All strains are derived from strain CNRZ1066. ^{a}Km, Spec, Cm and Erm = resistance to kanamycin, spectinomycin, chloramphenicol and erythromycin, respectively. ^{b}The arrows indicate construction via transformation with chromosomal DNA or a plasmid.**

| **Strain** | **Genotype** | **Resistance^{a}** | **Description^{b}** |
|---|---|---|---|
| TIL1523 | Δ*sepM*::*spec* | Spec | PCR fragment *sepM::spec* → CNRZ1066 |
| TIL1524 | *blp::shp-rgg₁₃₅₈*-Pₛₜₑᵣ*₁₃₅₇-luxAB-aphA3* | Km | pGICB004a::*shp-rgg₁₃₅₈*-P_{ster*1357*} → CNRZ1066 |
| TIL1525 | *blp::rgg₁₃₅₈*-P_{ster*1357*}-*luxAB-aphA3* | Km | pGICB004a::*rgg₁₃₅₈*-P_{ster*1357*}→ CNRZ1066 |
| TIL1535 | Δ*htrA*::*aphA3* | Km | PCR fragment *htrA*::*aphA3* → CNRZ1066 |
| TIL1536 | Δ*htrA*::*aphA3* Δ*sepM*::*spec* | Km Spec | TIL1535 DNA → TIL1523 |
| TIL1551 | *blp::shp-rgg₁₃₅₈*-Pₛₜₑᵣ*₁₃₅₇-elafin-P32cm* | Km Spec Cm | pEla::*shp-rgg₁₃₅₈*-P_{ster*1357*}→ TIL1536 |
| | Δ*htrA*::*aphA3* | | |
| | Δ*ywdF*::*spec* | | |
| TIL1552 | *blp::rgg₁₃₅₈*-Pₛₜₑᵣ*₁₃₅₇ elafin-P32cm* | Km Spec Cm | pEla::*rgg*_{*1*358}-P_{ster*1357*}→ TIL1536 |
| | Δ*htrA*::*aphA3* | | |
| | Δ*ywdF*::*spec* | | |
| TIL1566 | *blp::rgg₁₃₅₈*-Pₛₜₑᵣ*₁₃₅₇ luxAB-aphA3* | Km | PCR fragment *pptAB*::*erm* → TIL1525 |
| | Δ*pptAB::erm* | | |
| TIL1567 | *blp::rgg₁₃₅₈*-Pₛₜₑᵣ*₁₃₅₇ elafine- P32cm* | Km Erm | TIL1568 DNA → TIL1552 |
| | Δ*htrA*::*aphA3* | | |
| | Δ*ywdF*::*spec* | | |
| | Δ*pptAB::erm* | | |
| TIL1568 | Δ*pptAB::erm* | Erm | PCR fragment *pptAB::erm* → CNRZ1066 |

### DNA manipulation and sequencing

Restriction enzymes, T4 DNA ligase (New England Biolabs) and Phusion DNA polymerase (Finnzymes) were used in accordance with the manufacturers' instructions. Standard methods were used to carry out DNA purification, restriction digestion, PCR, ligation, and sequencing. *S*. *thermophilus* strain CNRZ1066 or its derivatives were transformed using natural competent cells (Gardan et al., J. Bacteriol. 191:4647-4655, 2009) prepared with the addition of synthetic competence peptide (ComS, LPYFAGCL (SEQ ID NO: 45) at a concentration of 1 µM. *L. lactis* electrocompetent cells were prepared and transformed as described previously (Holo H & Nes IF, Appl. Environ. Microbiol. 55:3119-3123, 1989). The plasmids used are listed in Table 6.

**Table 6. Plasmids. ^{a}Erm, Km and Cm = resistance to erythromycin, kanamycin and chloramphenicol, respectively.**

| **Plasmid** | **Description^{a}** |
|---|---|
| pGICB004a | Erm,Km, pG⁺host9 derivative containing the *luxAB* genes of *Photorhabdus luminescens* and an kanamycin resistance cassette surrounded by two fragments of the *blp* operon, allowing double crossover integration at the *blp* locus of *S*. *thermophilus* |
| pGICB004a::*shp-rgg₁₃₅₈*-P_{ster*1357*} | Erm, Km, pGICB004a derivative used to introduce a *shp-rgg₁₃₅₈*-P_{ster*1357*}-*luxAB* transcriptional fusion at the *blp* locus of *S*. *thermophilus* |
| pGICB004a::*rgg₁₃₅₈*-P_{ster*1357*} | Erm, Km, pGICB004a derivative used to introduce a *rgg₁₃₅₈*-Pₛₜₑᵣ*₁₃₅₇-luxAB* transcriptional fusion at the *blp* locus of *S*. *thermophilus* |
| pEla::*shp-rgg₁₃₅₈*-P_{ster*1357*} | Erm, Cm, pGICB004a derivative used to introduce a *shp-rgg₁₃₅₈*-P_{ster*1357*}-*elafin* transcriptional fusion at the *blp* locus of *S*. *thermophilus* |
| *pEla*::*rgg₁₃₅₈*-P_{ster*1357*} | Erm, Cm, pGICB004a derivative used to introduce a *rgg₁₃₅₈*-Pₛₜₑᵣ*₁₃₅₇-elafin* transcriptional fusion at the *blp* locus of *S*. *thermophilus* |

### Construction of the mutant strains

The overlapping PCR method was used to delete the *sepM* (STR_RS07745) and *htrA* (STR_RS09505) genes in strain CNRZ1066 and replace them with a spectinomycin (*spec*) and a kanamycin cassette (*aphA3*), respectively as previously described (Gardan *et al.,* 2009). Briefly, the *spec* and the *aphA3* cassettes were amplified via PCR using pAT28 and pKa plasmids respectively, as DNA template. Upstream and downstream fragments of the *sepM* and *htrA* genes were amplified using chromosomal DNA from strain CNRZ1066 as a template. The upstream fragments, the cassettes and the downstream fragments were fused by overlapping PCR. The resulting PCR fragments were used to transform strain CNRZ1066 leading to the construction of strain TIL1523 (*sepM::spec*) and strain TIL1535 (*htrA*::*aphA3*). The TIL1536 (*sepM::spec htrA*::*aphA3*) was constructed by transforming strain TIL1523 with chromosomal DNA from strain TIL1535. The same method was used to delete the *pptAB* genes (STR_RS07560-STR_RS07565) and replace them with an erythromycin cassette. The *erm* cassette was amplified via PCR using pG+host9 plasmid as a template. The protocol was the same except that chromosomal DNA of strain CNRZ1066 was used for the amplification of the upstream and downstream fragments. The resulting product of the overlapping PCR was used to transform strain CNRZ1066 and strain TIL1525 leading to the construction of strain TIL1568 (*ΔpptAB*::*erm*) and strain TIL1566 (*blp*::*rgg1358*-Pₛₜₑᵣ₁₃₅₇*luxAB ΔpptAB*::*erm*), respectively.

To study the relevance of two variants of the inducible expression system with the luciferase encoding genes, two derivatives of pGICB004a plasmid (Table 6), pGICB004a::*shp-rgg1358*-Pₛₜₑᵣ₁₃₅₇ (complete variant) and pGICB004a::*rgg1358*-Pₛₜₑᵣ₁₃₅₇ (incomplete variant), were constructed as follows. The *shp-rgg1358*-Pₛₜₑᵣ₁₃₅₇ fragment was amplified by PCR and chromosomal DNA from strain LMD-9 was used as a template; double digested with the restriction enzymes Spel and EcoRI; and finally ligated into pGICB004a between the related restriction sites leading to the construction of pGICB004a::*shp-rgg1358*-Pₛₜₑᵣ₁₃₅₇ plasmid. A similar approach was used for the construction of pGICB004a::*rgg1358*-Pₛₜₑᵣ₁₃₅₇. Both plasmids were linearized by Scal. Linearized plasmid pGICB004a::*shp-rgg1358*-Pₛₜₑᵣ₁₃₅₇ and pGICB004a::*rgg1358*-Pₛₜₑᵣ₁₃₅₇ were used to transform strain CNRZ1066 leading to strains TIL1524 (*blp*::*shp-rgg1358*-Pₛₜₑᵣ₁₃₅₇*luxAB*) and strain TIL1525 (*blp*::rgg1358-Pster1357-luxAB), respectively.

To study the functionality of the two variants of the inducible expression system with the production of elafin, two plasmids pEla::*shp-rgg1358*-Pₛₜₑᵣ₁₃₅₇ and pEla::*rgg1358*-Pₛₜₑᵣ₁₃₅₇ were constructed by replacing the *luxAB* genes and the *aphA3* cassette of plasmid pGICB004a::*shp-rgg1358*-Pₛₜₑᵣ₁₃₅₇ and pGICB004a::*rgg1358*-Pₛₜₑᵣ₁₃₅₇ with the elafin gene and a chloramphenicol cassette (P32Cm), as follows. The elafin gene fused to a signal peptide was constructed and amplified by PCR. The chloramphenicol cassette was amplified by PCR from plasmid pNZ5319. The two fragments were joined by overlapping PCR, double digested with the restriction enzymes Sall and EcoRI; and finally ligated into pGICB004a::*shp-rgg1358*-Pₛₜₑᵣ₁₃₅₇ and pGICB004a::*rgg1358-*Pₛₜₑᵣ₁₃₅₇ between the related restriction sites leading to the construction of pEla::*shp-rgg1358-*Pₛₜₑᵣ₁₃₅₇ and pEla::*rgg1358*-Pₛₜₑᵣ₁₃₅₇. Both plasmids were linearized by Scal. Linearized plasmid pEla::*shp-rgg1358*-Pₛₜₑᵣ₁₃₅₇ and plasmid pGICB004a::*rgg1358*-Pₛₜₑᵣ₁₃₅₇ were used to transform strain TIL1536 (*ΔhtrA::αphA3 ΔsepM::spec*) leading to strain TIL1551 (*blp*::*shp-rgg1358*-Pₛₜₑᵣ₁₃₅₇-*elafin-P32cm ΔhtrA::aphA3 ΔywdF::spec*) and TIL1552 (*blp::rgg1358*-Pₛₜₑᵣ₁₃₅₇*luxAB ΔhtrA::aphA3 ΔywdF::spec*), respectively. Chromosomal DNA of strain TIL1568 (*ΔpptAB::erm*) was used to transform strain TIL1552 leading to strain TIL1567 strain (*blp::rgg1358*-Pₛₜₑᵣ₁₃₅₇*-elafin-P32cm ΔhtrA::aphA3 ΔywdF::spec ΔpptAB::erm*). All constructions were verified by PCR and sequenced when necessary.

### Luciferase assays

Cells were grown overnight at 42°C in CDM. These cultures were then diluted in 50 ml of CDM to a final OD600 of 0.05 and incubated at 42°C. Aliquots of 1 ml of the culture were sampled at regular intervals until the culture reached stationary phase and analyzed as follows: OD600 was measured with 1 ml of culture, then 10 µl of a 0.1% nonyl-aldehyde solution was added and the luminescence was immediately measured with a Junior LB9509 (Berthold technologies). Induction of the expression of the luciferase encoding genes in strain TIL1525 was performed with the addition of SHP peptide (EGIIVIVVG, SEQ ID NO: 24) in the growth medium at a final concentration of 1 µM two hours after the beginning of the culture. Results were reported in Relative Luminescent Units divided by the OD600 (RLU / OD600).

### Western blots

For the preparation of the samples, derivatives of strain CNRZ1066 were grown in CDM at 42°C. When necessary, induction was performed with the addition of SHP peptide (EGIIVIVVG, SEQ ID NO: 24) at a final concentration of 1 µM for strain TIL1552 and 0.5 µM for strain TIL1567 in the growth medium at OD600 of 0.2. When the cultures reached the desired OD600, 10 ml were centrifuged at 3200 g for 10 min at room temperature. Filtered supernatants (0.22 µM, Millex GV PVDF, Millipore) were further ultrafiltered through Amicon devices (Ultra-15 [3-kDA cutoff]; Millipore) for 1 hour at 25°C at 3200g. Five hundred µl containing the secreted elafin were recovered in the upper compartment of the Amicon device.

For the Western blot experiments, 10 µl of the samples were diluted in Laemmli Buffer (4X) and heated to 95°C for 5 minutes. Samples were run on Precast Bis-Tris Gel 4-12% (NuPAGE 1.5mm X 10; Invitrogen) and transferred to nitrocellulose membranes (Trans-Blot turbo 0.2 µm PVDF; Biorad). The membrane were blocked two hours at room temperature in a TBST solution (Tris 0.1M pH 7.5, NaCl 1.5 M, Tween 20 1%) containing 5% skimmed milk and then hybridized for 2 hours at room temperature in TBST solution with anti-elafin antibody (Santa Cruz sc-398075, 1/1000 dilution). Four washing steps were performed in TBST solution, one for 15 min and three for 5 min and detection was achieved using secondary antibody coupled to peroxidase (Abliance BI2413C, 1/1000 dilution) during 1 hour. Four washing steps were again performed as previously described before the addition of a solution containing luminol (ECL prime, GE healthcare) for 5 min. Five µl of elafin (RD system, 1/100 dilution) were used as a control. Detection was finally recorded using a Chemidoc (Biorad).

### EXAMPLE I. The SHP/Rgg₁₃₅₈ system of S. thermophilus strain LMD-9 can be used as an expression system for the synthesis of luciferase

The *S*. *thermophilus* pangenome contains several SHP/Rgg systems. Some strains have accumulated up to 6 systems, like strain LMD-9. Among these different SHP/Rgg systems, SHP/Rgg₁₃₅₈ (comprising the promoter sequences SEQ ID NO: 18 and SEQ ID NO: 20 and the sequences encoding the Rgg protein of SEQ ID NO: 6 and the mature SHP of SEQ ID NO: 24) is the most well-known (Fleuchot et al., 2011). The *rgg* gene is annotated STER_RS06695 in NCBI but was designed as *rgg*₁₃₅₈ in previous publications based on a primary annotation of strain LMD-9 (Makarova *et al.,* 2006). The *shp* gene is not annotated but is located in front of the *rgg*₁₃₅₈ gene in a divergent orientation. This gene encodes a 24 amino acid peptide (MKKQILLTLLLVVFEGIIVIVVG, SEQ ID NO: 33) which is matured by cleavage between the phenylalanine and the glutamic acid residues by the membrane protease Eep and secreted in the extracellular medium by the transporter PptAB before being reimported by the Ami oligopeptide permease. Once inside the cellular compartment, the mature SHP interacts with Rgg₁₃₅₈ to positively control the expression of the *shp* gene itself and of a polycistronic operon whose the three first genes are involved in the production and secretion of a cyclic peptide called streptide (Figure 1A). The first gene (*ster_1357*) encodes the peptide that is further cyclized by a SAM radical enzyme encoded by the second gene (*ster_1356*) and exported by an exporter encoded by the third gene (*ster_1355*)*.* The function of the last three genes is still unknown. The control of the expression of the *shp* gene by the SHP/Rgg₁₃₅₈ complex creates a positive feedback loop that triggers the expression of both targets (the *shp* genes and the operon of six genes) suddenly at the middle of the exponential growth phase. When the *shp* gene of the system was deleted, the addition of synthetic mature SHP in the culture medium restored the expression of the *shp* gene in a dose response manner (Fleuchot *et al.,* 2011). The DNA sequence recognized by the complex SHP/Rgg₁₃₅₈ has been identified and is located upstream of the *shp* gene and of the streptide encoding gene (*ster_1357*)*.* This locus was chosen to test the relevance of its use as a new expression system. The strain CNRZ1066 was chosen as it does not naturally express this system. To do so, the luciferase was chosen as a reporter to easily measure the level of expression of our system.

Two variants were assessed (Figure 1B). The first one was made of the *shp* gene, the *rgg*₁₃₅₈ gene and the promoter of the streptide operon (P₁₃₅₇). This module is complete and is expected to trigger the expression of a gene of interest from the streptide promoter at the middle of the exponential growth phase. The second one lacks the *shp* gene and is designed as incomplete. The expression of the gene of interest must be triggered by the addition of exogenous mature SHP in the growth medium. Both DNA fragments, complete (*shp*-*rgg*₁₃₅₈-P1357) and incomplete (*rgg*₁₃₅₈-P1357), were amplified by PCR from chromosomal DNA of strain LMD-9 and cloned upstream of the *luxAB* reporter genes of plasmid pGICB004a creating plasmids pGICB004a::*shp-rgg1358*-Pₛₜₑᵣ₁₃₅₇ and pGICB004a::*rgg*₁₃₅₈-Pₛₜₑᵣ₁₃₅₇. Plasmid vector pGICB004a allows the integration of transcriptional fusions at the *blp* locus of *S*. *thermophilus* strains after a double recombination event. The luciferase reporter strain carrying the complete variant was named TIL1524 and the one carrying the incomplete variant, TIL1525. As expected, strain TIL1524 (*blp*::*shp-rgg*₁₃₅₈-Pₛₜₑᵣ₁₃₅₇-*luxAB*) grown in CDM showed a triggering of the expression of the fusion at the middle of the exponential growth phase with a peak reached at the end of this exponential phase with 4 10⁶ RLU/OD₆₀₀ (Figure 2A). Strain TIL1525 (*blp*::*rgg*₁₃₅₈-Pₛₜₑᵣ₁₃₅₇-*luxAB*) was grown in two different media, CDM and CDM in which synthetic SHP was added after 2 hours of growth (Figure 2B). The addition of SHP in the growth medium triggered a rapid expression of the luciferase genes and the peak was reached at the end of the exponential growth phase to a similar level that the one obtained with strain TIL1524 (3 10⁶ RLU/OD₆₀₀). However, when SHP was omitted, we also observed a triggering of the expression of the luciferase genes but the level of the peak was two times lower than the one obtained with SHP. Although the CNRZ1066 strain genome does not encode a SHP/Rgg₁₃₅₈ system, it encodes 5 other SHP/Rgg systems with some mature SHP amino acid sequences highly similar to the one of the SHP₁₃₅₈ (Table 7).

**Table 7. Amino acid sequences of the SHP found in S. thermophilus strain CNRZ1066. The amino acid sequence of the mature form of the SHP of inducible system of strain LMD-9 is EGIIVIVVG (SEQ ID NO: 24). *The predicted sequence of the mature form is in bold.**

| Locus tag of the *rgg* gene | Amino acid sequence of the corresponding SHP* |
|---|---|
| STR_RS07375 | MKKQKLLLLVVLVC**EGIIVILVG** (SEQ ID NO: 34) |
| STR_RS09145 | MNKKALFSLLFVIL**EGIIVIGVG** (SEQ ID NO: 35) |
| STR_RS04530 | MNKESFLAILLLIF**ESIIVIAVG** (SEQ ID NO: 36) |
| STR_RS01100 | MKLLKIIVLLT**CIYTIVGGV** (SEQ ID NO: 40) |
| STR_RS05050 | MEKVSKILPILILVM**DIIIIVGG** (SEQ ID NO : 38) |

It is highly probable that in strain TIL1525, one (or more) of these SHPs interacts with the Rgg₁₃₅₈ of strain LMD-9 to control its activity. To confirm this hypothesis, we introduced the Δ*pptAB*::*erm* in strain TIL1525 (*blp*::*rgg*₁₃₅₈-Pₛₜₑᵣ₁₃₅₇-*luxAB*) creating strain TIL1566. In a Δ*pptAB*::*erm* mutant, none of the SHPs encoded by CNRZ1066 genome can be exported. The positive feedback loops at the origin of the production of the SHPs is blocked and consequently, the SHPs are not synthesized. Indeed, in strain TIL1566, no luminescence could be measured (Figure 2C), confirming the hypothesis that the leakiness of the promoter P₁₃₅₇ of strain TIL1525 is totally corrected with the addition of the Δ*pptAB::erm* mutation in strain TIL1566.

### EXAMPLE II. The SHP/Rgg₁₃₅₈ system can be used as an expression system for the synthesis of secreted heterologous protein, the elafin

The elafin protein is a protease inhibitor of human origin found in the gut and known to have a protective effect against inflammatory bowel disease. The gene encoding this protein was chosen to test the relevance of the inducible system for the production of a secreted protein with therapeutic properties. For that purpose, the *luxAB* genes and the *aphA3* kanamycin cassette of the two plasmids pGICB004a::*shp*-*rgg*₁₃₅₈-Pₛₜₑᵣ₁₃₅₇ and pGICB004a::*rgg*₁₃₅₈-Pₛₜₑᵣ₁₃₅₇ were replaced with the elafin gene fused downstream of a DNA fragment encoding the secretion signal peptide of USP45, the main secreted protein of *L. lactis* and a P32Cm cassette conferring resistance to chloramphenicol (Figure 1B). Both resulting plasmids were linearized and used to transform strain TIL1536 (*ΔhtrA*::*aphA3* Δ*sepM*::*spec*). This strain devoid of two major extracellular proteases has a reduced surface proteolytic activity and reduces the degradation of secreted proteins (PCT/EP2021/055561). The two constructed strains TIL1551 (*blp::shp-rgg₁₃₅₃*-Pₛₜₑᵣ*₁₃₅₇-elafin-P32cm* Δ*htrA*::*aphA3* Δ*ywdF*::*spec*) and TIL1552 (*blp::rgg₁₃₅₃*-Pₛₜₑᵣ*₁₃₅₇-elafin-P32cm* Δ*htrA*::*αphA3* Δ*ywdF*::*spec*) harboring the complete and incomplete variants of the inducible system, respectively, were grown in CDM. Strain TIL1536, which does not contain the elafin gene in his genome, was used as a negative control and grown in CDM, as well. As expected, no trace of elafin could be detected in any sample prepared from this strain (Figures 3A and B). With strain TIL1552, induction of the system was performed with the addition of SHP at OD₆₀₀ 0.2. Samples of supernatant were recovered at different OD₆₀₀ and used to detect the presence of elafin by Western blot analysis as described in the materials and methods section. In the supernatant of strain TIL1551 containing the complete variant, elafin could not be detected at OD₆₀₀ 0.2, but was detected at OD₆₀₀ 0.5 (Figure 3A) and at OD₆₀₀ 1 (Figure 3B). In the supernatant of strain TIL1552, without induction, the elafin was not detected at OD 0.2, started to be slightly detected at OD₆₀₀ 0.5 (Figure 3A) and was clearly detected at OD₆₀₀ 1 and 2 (Figure 3B). In the induced culture of strain 1552, elafin was detected at OD₆₀₀ 0.5 at a level much greater than the one obtained with the uninduced culture (Figure 3A). This difference between the induced and uninduced cultures was less pronounced at OD₆₀₀ 1 and 2 (Figure 3B). At OD₆₀₀ 2, a fainted band corresponding to a degradation fragment of the elafin was also detected in the induced and uninduced cultures (Figure 3B). These results clearly highlight that the induction performed at OD₆₀₀ 0.2 is efficiently detected at OD₆₀₀ 0.5 at middle of the exponential phase of growth but also confirm the leakiness of the inducible promoter with the detection of elafin in the uninduced culture at OD₆₀₀ 0.5. To overcome this drawback, as for the luciferase reporter strain, we introduced a Δ*pptAB*::*erm* mutation in strain TIL1552 creating strain TIL1567. As shown on Figure 3C, the elafin was not detected in the supernatant of strain TIL15567 in uninduced condition either at OD₆₀₀ 0.5 or 1. However, as expected, the production of elafin in the extracellular medium was restored with the addition of SHP in the culture medium and detected at OD₆₀₀

## Claims

1. A genetically modified bacterium capable of inducibly expressing a nucleic acid sequence of interest, wherein:
the genetically modified bacterium comprises a genetic construct comprising the nucleic acid sequence of interest operably linked to a promoter positively regulated by a protein complex formed by a small hydrophobic peptide called "SHP" and a regulator protein called "Rgg",
the promoter comprises a DNA-binding site selected from the group consisting of: GCA(A/T)ATA(T/G)GGGAAT(A/T) (SEQ ID NO: 1), AATTGC(G/T)TATAAGGGAAA (SEQ ID NO: 2), ATTTCATATCTTCAATTTT (SEQ ID NO: 3) and variants thereof having 1 or 2 substitutions,
the genetically modified bacterium expresses the Rgg protein,
the Rgg protein has at least 80% sequence identity with an amino acid sequence selected from the group consisting of: SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID: 11, SEQ ID NO: 12 and SEQ ID NO: 13.

2. The genetically modified bacterium according to claim 1, wherein the bacterium expresses a native form of the SHP peptide.

3. The genetically modified bacterium according to claim 1, wherein the bacterium does not express a native form of the SHP peptide.

4. The genetically modified bacterium according to any one of claims 1 to 3, wherein the genetic construct further comprises a nucleic sequence encoding the Rgg protein.

5. The genetically modified bacterium according to any one of claims 1, 2 or 4, wherein the genetic construct further comprises a nucleic sequence encoding the SHP peptide.

6. The genetically modified bacterium according to any one of claims 1 to 5, wherein an ABC-type transporter called "PptAB", which enables export of the SHP peptide to the extracellular medium, is not functional or is inactivated.

7. The genetically modified bacterium according to any one of claims 1 to 6, wherein a membrane protease called "Eep", which enables cleavage of the native form of the SHP peptide, is not functional or is inactivated.

8. The genetically modified bacterium according to any one of claims 1 to 7, wherein the bacterium has no or low surface proteolytic activity.

9. A genetic construct comprising:
- a nucleic acid sequence of interest operably linked to a promoter that is positively regulated by a protein complex formed by a small hydrophobic peptide called "SHP" and a regulator protein called "Rgg", said promoter comprising a DNA-binding site selected from the group consisting of: GCA(A/T)ATA(T/G)GGGAAT(A/T) (SEQ ID NO: 1), AATTGC(G/T)TATAAGGGAAA (SEQ ID NO: 2), ATTTCATATCTTCAATTTT (SEQ ID NO: 3) and variants thereof having 1 or 2 substitutions, and at least one genetic element selected from the group consisting of:
- a nucleic acid sequence encoding the Rgg protein, said Rgg protein having at least 80% sequence identity with an amino acid sequence selected from the group consisting of: SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13, and
- a nucleic acid sequence encoding the SHP peptide, said SHP peptide being selected from the group consisting of: SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31 and SEQ ID NO: 32.

10. A genetic vector comprising a genetic construct according to claim 9.

11. Use of a genetically modified bacterium as defined in any one of claims 1 to 8, a genetic construct as defined in claim 9 or a genetic vector as defined in claim 10, for producing a protein of interest, said protein of interest being encoded by the nucleic acid sequence of interest.

12. Method for producing a protein of interest comprising a step of culturing a genetically modified bacterium as defined in any one of claims 1 to 8 or a bacterium transformed with a genetic construct as defined in claim 9 or a genetic vector as defined in claim 10.

13. Method according to claim 12, wherein said genetically modified bacterium is cultivated in a culture medium supplemented with the SHP peptide, preferably during the exponential phase of growth.

14. A genetic vector for inducibly expressing a nucleic acid sequence of interest comprising:
- a promoter that is positively regulated by a protein complex formed by a small hydrophobic peptide called "SHP" and a regulator protein called "Rgg", said promoter comprising a DNA-binding site selected from the group consisting of: GCA(A/T)ATA(T/G)GGGAAT(A/T) (SEQ ID NO: 1), AATTGC(G/T)TATAAGGGAAA (SEQ ID NO: 2), ATTTCATATCTTCAATTTT (SEQ ID NO: 3) and variants thereof having 1 or 2 substitutions, and being located in 5' of a cloning site allowing the insertion of the nucleic acid sequence of interest,
and at least one genetic element selected from the group consisting of:
- a nucleic acid sequence encoding the Rgg protein, said Rgg protein having at least 80% sequence identity with an amino acid sequence selected from the group consisting of: SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13, and
- a nucleic acid sequence encoding the SHP peptide, said SHP peptide being selected from the group consisting of: SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31 and SEQ ID NO: 32.

15. Kit for inducibly expressing a nucleic acid sequence of interest comprising:
- a genetic vector as defined in claim 14, and at least one component selected from:
- a SHP peptide, and
- a bacterium, preferably a *Streptococcus.*
